# EUROPEAN PATENT SPECIFICATION

(11) **EP 4 100 093 B1**
(45) Date of publication and mention of the grant of the patent: **14.10.2026**
(21) Application number: 20917983.7
(22) Date of filing: 10.12.2020
(51) Int. Cl.: A61M 25/02, A61B 90/10, A61B 17/02

(54) **SYSTEMS AND METHODS FOR A CATHETER STABILIZATION AND OCCLUDER APPARATUS**
SYSTEME UND VERFAHREN FÜR EINE KATHETERSTABILISIERUNGS- UND OKKLUSIONSVORRICHTUNG
SYSTÈMES ET PROCÉDÉS POUR UN APPAREIL DE STABILISATION ET D'OCCLUSION DE CATHÉTER

(30) Priority: 04.02.2020 US 202062969711 P; 19.02.2020 US 202062978492 P
(43) Date of publication of application: 14.12.2022
(73) Proprietor: Dignity Health, San Francisco, CA 94107 (US)
(72) Inventor: COLE, Tyler, San Francisco, California 94107 (US); GRAHAM, Dakota, San Francisco, California 94107 (US); BOHL, Michael, San Francisco, California 94107 (US)
(74) Representative: Hutter, Anton
(86) International application number: PCT/US2020/064305
(87) International publication number: WO 2021/158284

(56) References cited:
- EP-A2- 1 048 320
- CN-A- 110 404 149
- DE-C1- 19 749 741
- US-A1- 2006 058 738
- US-A1- 2007 142 784
- US-A1- 2007 249 980
- US-A1- 2007 249 980
- US-A1- 2014 155 859
- US-A1- 2015 119 808
- US-A1- 2018 311 007

## Description

### CROSS-REFERENCE TO RELATION APPLICATIONS

The present application claims priority under 35 U.S.C. § 119 to U.S. Provisional Patent Application No. 62/969,711; filed February 4, 2020 and U.S. Provisional Patent Application No. 62/978,4492; filed February 19, 2020.

### FIELD

The present disclosure generally relates to medical devices; and in particular, to an apparatus and associated method for stabilizing a catheter during a surgical procedure.

### BACKGROUND

During surgical placement of a catheter in the body, it is often necessary to perform other aspects of a surgery, while also ensuring that the catheter remains stabilized or otherwise held in place. In many surgical applications, this may be frequently done using an extra "set of hands" in the operating room. During surgery, it is also sometimes necessary to occlude, or pinch shut, a catheter while the catheter is in position. For example, during placement of a valve, the catheter has to be stabilized and occluded first in order to safely and securely position the valve within the body near the catheter before securing the catheter to the valve. The task of stabilizing and occluding the catheter is often cumbersome and requires at least one extra pair of hands to occlude and stabilize the catheter. Further, current retractor devices such as metal retractors commonly used in surgical applications, are not compatible with electromagnetic (EM) neuronavigation as they are typically comprised of ferromagnetic materials that interfere. During many shunt procedures that use EM neuronavigation, a typical metal retractor would need to be removed during navigation and placed again after navigation.

EP 1 048 320 A2 describes an anchoring system for securing elongated tubes using pivotally connected plates.

It is with these observations in mind, among others, that various aspects of the present disclosure were conceived and developed.

### BRIEF DESCRIPTION OF THE DRAWINGS

FIG. **1** is a perspective view showing a first embodiment of a catheter stabilizer defining a retention body and an occluding arm;
FIG. **2** is a perspective view showing the catheter stabilizer of FIG. **1** showing the occluding arm in an "occluding" position;
FIG. **3** is a perspective view showing the catheter stabilizer of FIG. **1** with the occluding arm removed;
FIG. **4** is a perspective view showing the occluding arm of the catheter stabilizer of FIG. **1****;**
FIG. **5** is a perspective view showing a second embodiment of a catheter stabilizer defining a retention body and an occluding arm;
FIG. **6** is a perspective view showing the catheter stabilizer of FIG. **5** with the occluding arm removed;
FIG. **7** is a rear perspective view showing the catheter stabilizer of FIG. **5** with the occluding arm removed;
FIG. **8** is a top view showing the catheter stabilizer of FIG. **5** in a "folded" configuration;
FIG. **9** is a perspective view showing a third embodiment of a catheter stabilizer defining a retention body and an occluding arm;
FIG. **10** is a top view showing the catheter stabilizer of FIG. **9****;**
FIG. **11** is an exploded view showing the catheter stabilizer of FIG. **9**;
FIG. **12** is a perspective view showing a fourth embodiment of a catheter stabilizer;
FIG. **13** is a perspective view showing the catheter stabilizer of FIG. **12** having a clamping occluding arm;
FIG. **14** is a perspective view showing a fifth embodiment of a catheter stabilizer;
FIG. **15** is a front perspective view showing a sixth embodiment of a catheter stabilizer;
FIG. **16** is a rear perspective view showing the catheter stabilizer of FIG. **15****;**
FIG. **17A** is a first sequential view showing a screw being engaged with the retention body of the catheter stabilizer of FIG. **1** prior to insertion within an incision;
FIG. **17B** is a second sequential view showing the catheter stabilizer of FIG. **1** being positioned over a burr hole in a cranium of a patient;
FIG. **17C** is a third sequential view showing the catheter stabilizer of FIG. **1** being secured onto a cranium of a patient;
FIG. **17D** is a fourth sequential view showing insertion of a ventricular catheter into the occluding arm of the catheter stabilizer of FIG. **1****;**
FIG. **17E** is a fifth sequential view showing the occluding arm of the catheter stabilizer of FIG. **1** retaining the ventricular catheter within the burr hole;
FIG. **17F** is a sixth sequential view showing the occluding arm of the catheter stabilizer of FIG. **1** and the ventricular catheter of FIG. **17E** having been cut;
FIG. **17G** is a sixth sequential view showing the ventricular catheter of FIG. **17E** being removed from the occluding arm of the catheter stabilizer of FIG. **1** to demonstrate an efficacy of the occluding arm, visible by virtue of previously-occluded fluid exiting the catheter;
FIG. **17H** is a seventh sequential view showing placement of a valve caudal to the catheter while the catheter stabilizer of FIG. **1** holds the catheter in place and retains its position above the burr hole (note that the metal retractor is removed);
FIG. **17I** is an eighth sequential view showing the catheter being engaged with the valve while the catheter stabilizer of FIG. **1** holds the catheter in place and retains its position above the burr hole;
FIG. **17J** is a ninth sequential view showing removal of the screw of the catheter stabilizer of FIG. **1****;**
FIG. **17K** is a tenth sequential view showing removal of the catheter stabilizer of FIG. **1** from the burr hole;
FIG. **18** is a diagram showing an illustrative non-claimed method of insertion of the catheter stabilizer; and
FIG. **19** is a diagram showing an illustrative non-claimed method of removal of the catheter stabilizer.

Corresponding reference characters indicate corresponding elements among the view of the drawings. The headings used in the figures do not limit the scope of the claims.

### DETAILED DESCRIPTION

The invention is defined in independent claim 1. Further aspects are defined in the dependent claims 2-14. Methods of use described herein are not part of the claimed invention and left for illustrative purposes.

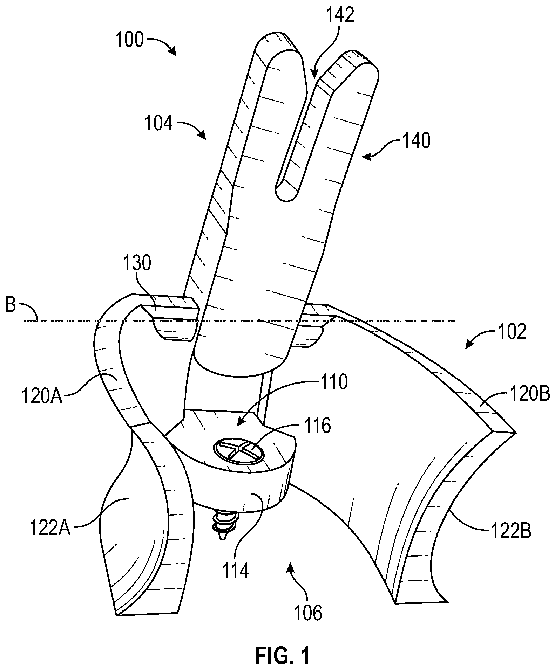

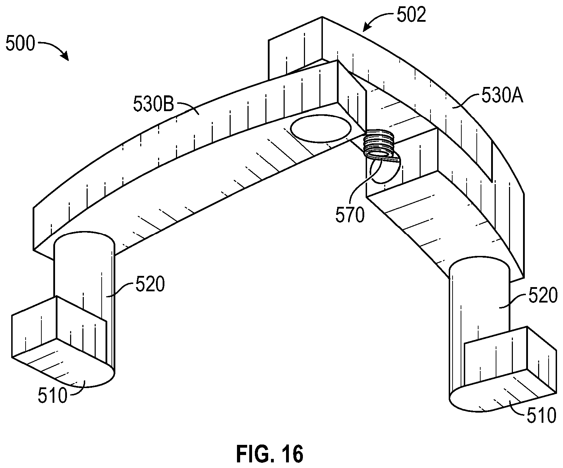

Various embodiments of a catheter stabilizer apparatus and associated method for stabilizing a catheter within a cranial burr hole are disclosed herein. In a primary embodiment, the catheter stabilizer is configured to be positioned and secured over a burr hole bored in a cranium of a patient during surgery. In some embodiments, the catheter stabilizer includes a retention body including one or more retention members for exposing the burr hole and retracting back cranial tissue of an incision to allow a clear workspace. The retention body further defines a channel configured to be oriented above the burr hole; in some embodiments the channel can provide reference for drilling a burr hole after placement of the retention body. The catheter stabilizer further includes an occluding arm, which can be positioned across an opening of the burr hole for receiving and occluding a catheter within the burr hole. Alternatively, the occluding arm may be positioned away from the burr hole to expose the channel and allow the surgeon to work within the burr hole. In some embodiments, the retention body is configured to be secured to the cranium of the patient. In one method of installation, the catheter stabilizer is inserted into an incision positioned over the burr hole and secured to the cranium. The occluding arm is then lowered into an "occlusion" position and the catheter inserted into a slot of the occluding arm. In some embodiments, the catheter stabilizer apparatus can be used for retracting cranial tissue and stabilizing/occluding catheters during new shunt placement and shunt revision procedures. Referring to the drawings, embodiments of a catheter stabilizer are illustrated and generally indicated as **100** in FIGS. **1-19****.**

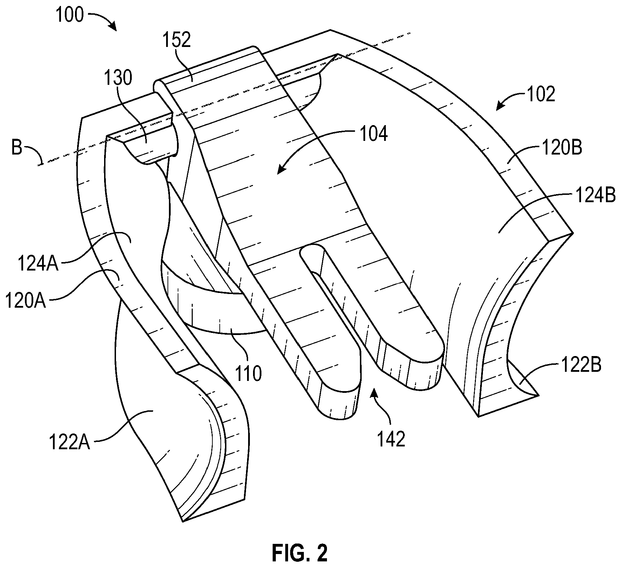

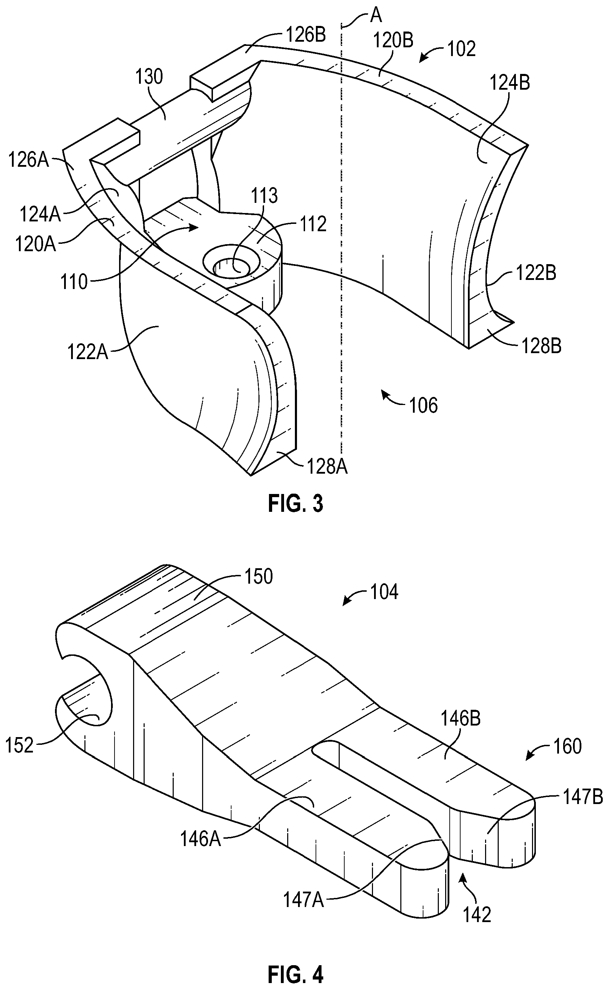

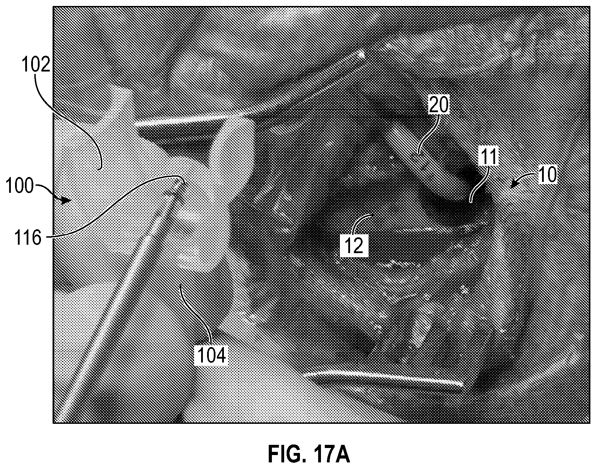

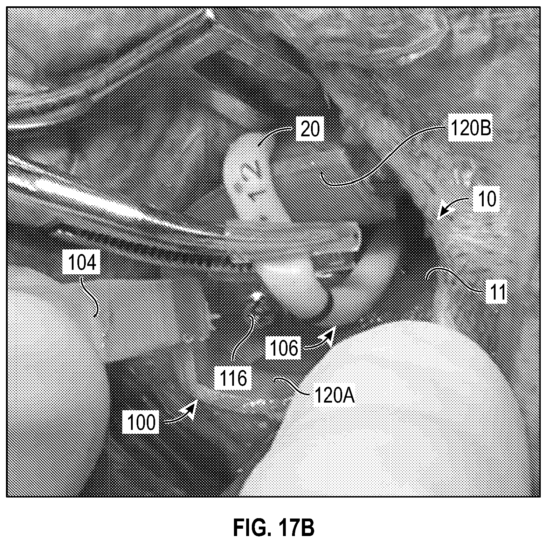

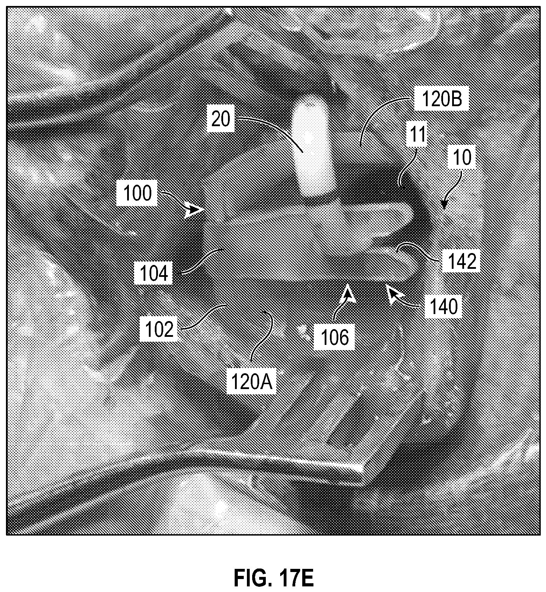

Referring to FIGS. **1-4** and **17A-K,** the catheter stabilizer **100** includes a retention body **102** for insertion within an incision **10** (FIG. **17A**), the retention body **102** defining a channel **106** defining an axis **A** (FIG. **3**) for alignment with a burr hole **11.** The retention body **102** is configured to retract cranial tissue of the incision **10** to expose the burr hole **11.** In some embodiments, the retention body **102** is in operative association with an occluder arm **104,** the occluder arm **104** configured to be positioned across the channel **106** to receive and occlude a catheter **20.** As shown in FIG. **1****,** the retention body **102** further includes an engagement point **110** defining a perforation **113** (FIG. **3**) through a surface **112** configured to contact the cranium for insertion of a securing member **116** into the cranium. FIG. **1** shows occluder arm **104** of the catheter stabilizer **100** in an "open" configuration with the occluder arm **104** rotated about an axis **B** away from the channel **106.** In the "open" configuration of FIG. **1****,** the channel **106** and engagement point **110** are exposed. In FIG. **2****,** the occluding configuration is shown in an "occlusion" position with the occluder arm **104** rotated about the axis **B** such that the occluder arm **104** is positioned across the channel **106.** In operation, as shown in FIG. **17B****,** the catheter **20** is passed through the channel **106** of the catheter stabilizer **100.** As further shown, the occluder arm **104** defines a slot **142** associated with a free end **140** of the occluder arm **104** for insertion and occlusion of a catheter **20** (FIG. **17E**).

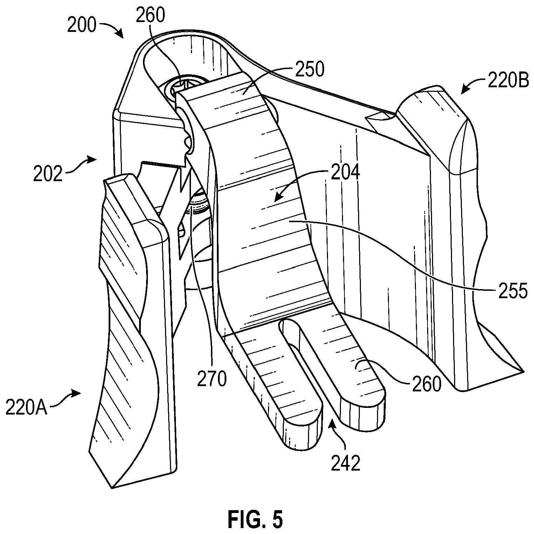

As discussed above, the retention body **102** includes one or more retention members **120;** in the embodiment shown in FIGS. **1-4** and **17A-K,** the retention body **102** defines a first retention member **120A** and an opposite second retention member **120B** that collectively form the channel **106,** as shown specifically in FIG. **3****.** In some embodiments, the retention members **120** are integral with the retention body **102,** as shown in the embodiment of FIGS. **1-4****,** and in other embodiments coupled to one another by a joint **260,** such as in the embodiment of FIGS. **5-8****.** As shown, the retention members **120A** and **120B** each define a proximal portion **126A** and **126B** and a distal portion **128A** and **128B,** respectively. In some embodiments, the retention members **120** each define a concave outer surface **122** for engagement with cranial tissue and an inner surface **124** that at least partially forms the channel **106.** In some embodiments, an underside (not shown) of the retention body **102** (and retention body **202** of embodiment **200** illustrated in FIG. **5**) also defines a generally concave surface to aid in conformity to the cranium. The retention body **102** further includes an engagement rod **130** defined between each proximal portion **126A** and **126B** of the first and second retention members **120A** and **120B** and configured to receive a hand **152** of the occluder arm **104.** The engagement rod **130** is located at a junction defined between the first and second retention members **120A** and **120B** and defines the axis **B** (FIG. **1**) that the occluding arm **104** is rotated around. In some embodiments, the catheter stabilizer **100** including the retention body **102** and the occluder **104,** as well as in other embodiments **200, 300, 400** and **500** described herein, are manufactured from a non-ferrous or non-ferromagnetic material such as medical-grade vinyl, plastic or another suitable material.

Referring to FIG. **4****,** the occluding arm **104** defines a proximal portion **150** and a distal portion **160.** The proximal portion **150** of the occluder arm **104** includes a hand **152,** which is configured to couple to the engagement rod **130** of the retention body **102,** as shown in FIGS. **1** and **2****.** The hand **152** allows the retention body **102** to be removably coupled to the occluder arm **104** and also allows the occluder arm **104** to be rotated about the axis **B** (FIG. **1**) between the open position (FIG. **1**) and the occluding position (FIG. **2**). In some embodiments, friction between respective surfaces of the engagement rod **130** of the retention body **102** and the hand **152** of the occluder arm **104** allows the occluder arm **104** to keep a stable position relative to the channel **106.** In other embodiments, the occluder arm **104** can snap between the open and occluding position.

The distal portion **160** of the occluding arm **104** defines a free end and includes a receptacle **142** for insertion and occlusion of the catheter **20.** As shown, the receptacle **142** has a slotted configuration and defines a first prong **146A** and a second prong **146B** collectively defining an opening **144** at the distal portion **160** of the occluding arm **104.** Each prong **146A** and **146B** includes a respective tapered shoulder portion **147** defined at the end of each prong **146** to guide the catheter **20** into the receptacle **142.** In some embodiments, a width of the receptacle **142** is sufficiently thin to allow the catheter **20** to deform and "pinch" shut when the catheter **20** is fully inserted into the receptacle **142.**

Referring to FIGS. 17A-K, 18 and **19,** surgical use of the catheter stabilizer **100** is described and illustrated herein. In particular, FIGS. **17A-17K** are photographs showing insertion of a ventriculo-atrial shunt during a shunt procedure aided by the catheter stabilizer **100** of FIGS. **1-4****.** The catheter stabilizer **100** secured a ventricular catheter **20** without assistance from a "second pair of hands" during the shunt procedure while other parts of the shunt procedure were being performed. During the procedure illustrated in FIGS. **17A-17K****,** a patient (not shown) was positioned in a supine position with a "bump" underneath a right shoulder and a head turned toward the left and their anatomy was registered to an electromagnetic (EM)-based neuronavigation system. It should be noted that the catheter stabilizer 100 allows retraction of cranial tissue and catheter occlusion without interfering with EM neuronavigation such as during use of a metal retractor. The shunt procedure included the following steps: (1) reopening a cranial incision **10** to expose the burr hole **11** drilled into the cranium; (2) placement of the catheter stabilizer **100** into the incision **10** and above the burr hole **11;** (3) placement of a transjugular atrial catheter; (4) connection of a ventricular catheter **20** to a valve and the atrial catheter; and (5) removal of the catheter stabilizer **100.** As shown in FIGS. **17A** and **17B****,** the catheter stabilizer **100** includes the retention body **102** and the occluder arm **104.** In operation, the occluder arm **104** is rotated away from the occluding position to expose the channel **106** (FIG. **17B**) and the engagement point **110** for insertion of the cranial securing member **116** into the perforation **113** (FIG. **3**) of the engagement point **110.** In the embodiment shown, the cranial securing member **116** is a 6mm self-tapping screw. After additional testing it was found that potential screw compatibility is 6-8mm but may vary depending on surgeon preference. FIG. **17A** also shows a pilot hole **12** drilled in the cranium adjacent to the burr hole **11.**

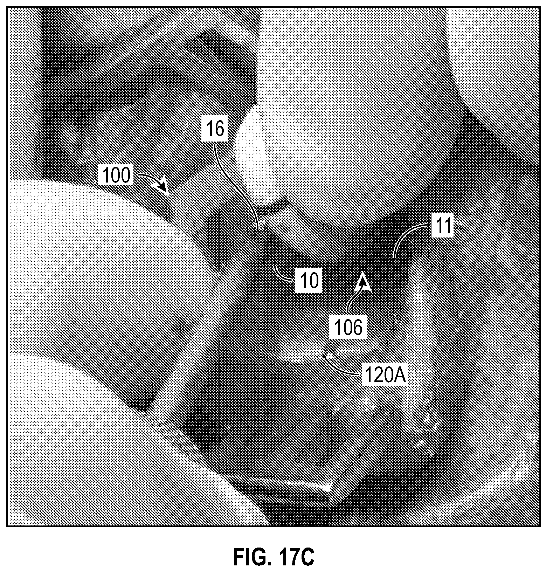

In FIG. **17B****,** the catheter stabilizer **100** was placed into the incision **10** above the burr hole **11.** The incision **10** was held open by a pair of retention members **122** and **124** before the catheter **20** was passed through the channel **106.** The cranial securing member **116** is shown being inserted into the pilot hole **12** (visible in FIG. **17A**) and the securing member **116** screwed into the pilot hole **12** of the cranium and tightened against the surface **112** of the engagement point **110** in FIG. **17C****.**

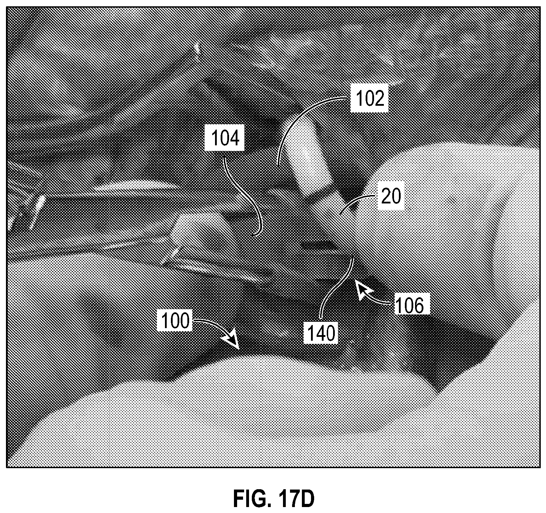

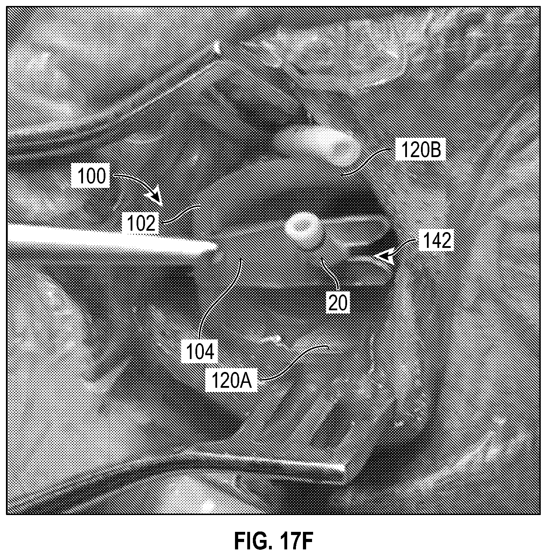

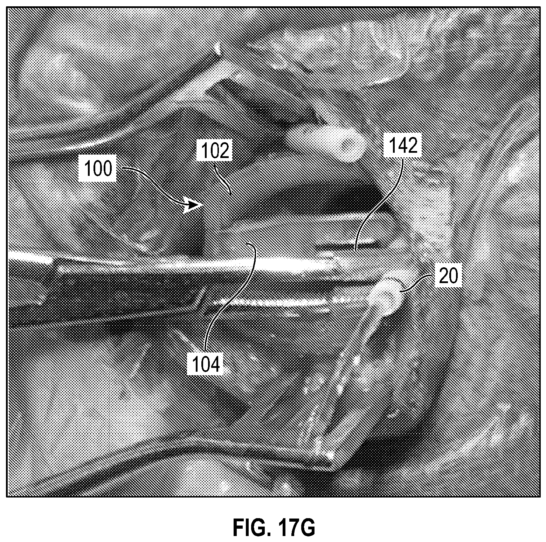

Referring to FIGS. **17D** and **17E****,** once the catheter stabilizer **100** was secured within the incision **10** and above the burr hole **11,** the occluder arm **104** was then rotated about axis **B** (FIG. **1**) to the "occluding" position such that the occluder arm **104** is positioned across the channel **106** and burr hole **11.** As shown specifically in FIG. **17D****,** the catheter **20** was inserted into the distal portion **140** (receptacle **142,** visible in FIG. **17E**) of the occluder arm **104.** In FIG. **17E****,** the catheter **20** was then captured by the receptacle **142** that communicates with the distal portion **140** of the occluder arm **104.** In FIG. **17F****,** the catheter stabilizer **100** is shown holding the catheter **20** in place within receptacle **142** while the catheter **20** is cut. In FIG. **17G****,** the catheter **20** is shown moved from the receptacle **142** of the occluder arm **104** with fluid flowing out of the catheter **20** when not occluded by the occluder arm **104,** demonstrating the efficacy of the catheter stabilizer **100** when occluding the catheter **20.**

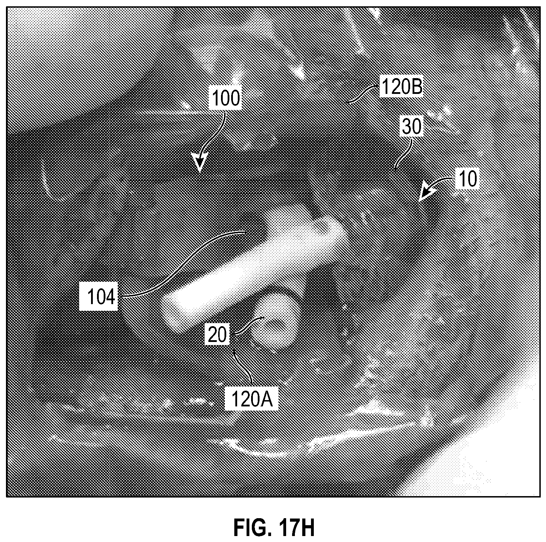

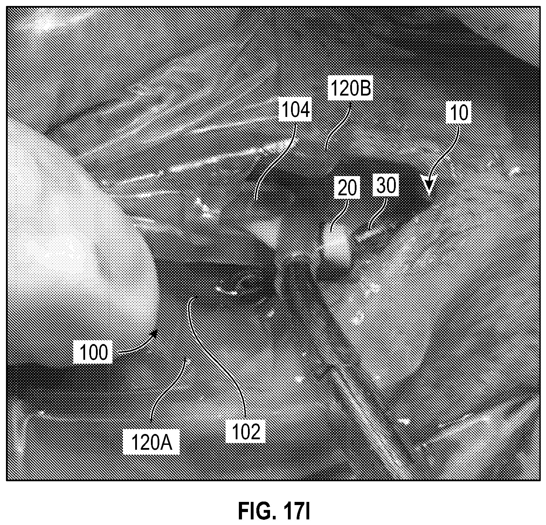

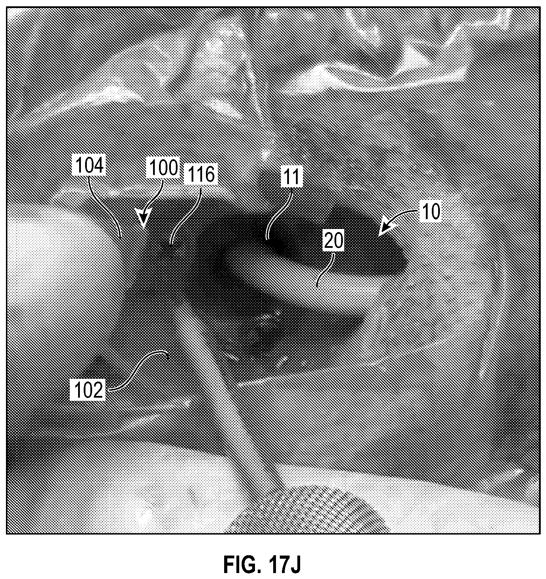

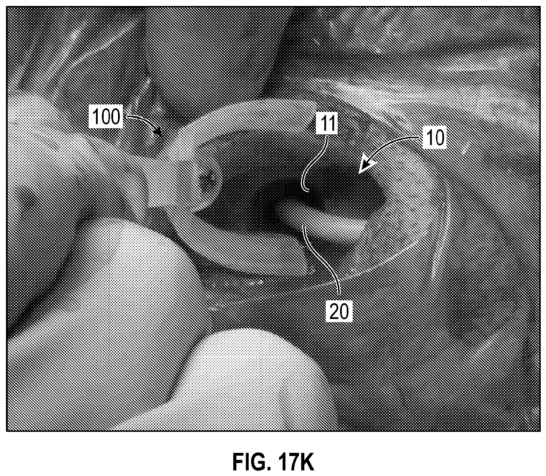

In FIGS. **17H** and **17I,** efficacy of the catheter stabilizer **100** when holding the catheter **20** in place during insertion of a valve **30** is demonstrated. During the test, the catheter **20** was held firmly in place and "pinched" shut by the occluder arm **104.** The catheter stabilizer **100** remained firmly inside the incision **10** above the burr hole **11** (not visible) during placement of the valve **30** and connection of the valve **30** with the catheter **20.** FIGS. **17H** and **17I** also indicate that the metal retractor was removed during the procedure while the incision **10** remained open and the ventricular catheter **20** remained occluded. The catheter stabilizer **100** does not interfere with the electromagnetic navigation used to identify the trajectory of the ventricular catheter **20.** In FIGS. **17J** and **17K****,** to remove the catheter stabilizer **100,** the catheter **20** was removed from the receptacle **142** (FIG. **17E****)** and the occluder arm **104** rotated about the horizontal axis **B** away from the "occluding" position to expose the securing member **116** of the engagement point **110.** In the embodiment shown, the securing member **116** is loosened from the pilot hole **12** and the retention body **102** of the catheter stabilizer **100** is removed from the incision **10.**

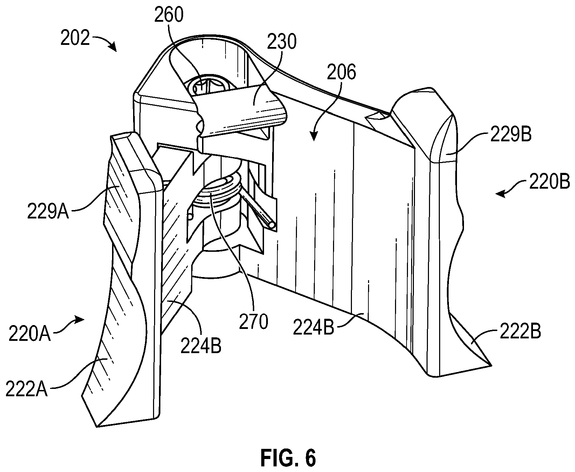

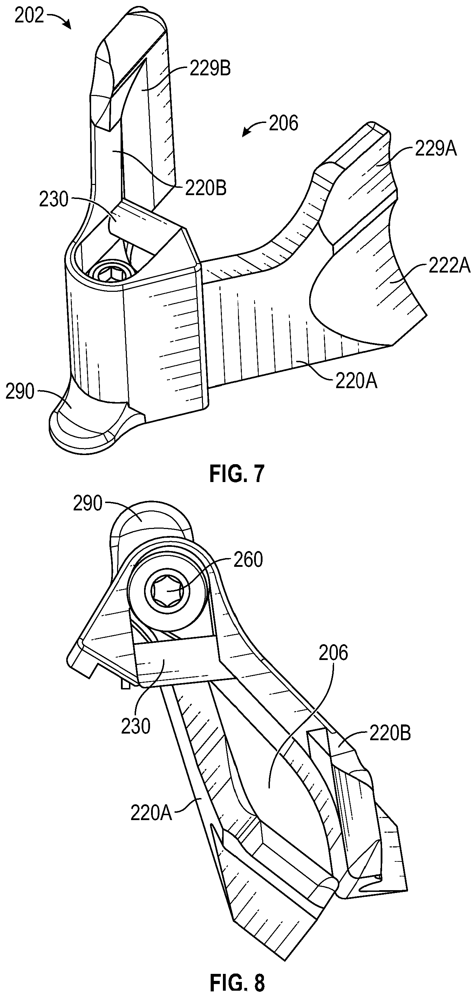

Referring to FIGS. **5-8****,** a second embodiment of a catheter stabilizer **200** is illustrated. Similar to the catheter stabilizer **100** of FIGS. **1-4****,** the catheter stabilizer **200** includes an occluding arm **204** and a retention body **202** for insertion and engagement within the incision **10** (FIG. **17A**) to retain cranial tissue away from the burr hole **11** (FIG. **17A**), the retention body **202** including first and second retention members **220A** and **220B.** As shown, in some embodiments the first and second retention members **220A** and **220B** are discrete from one another and are pivotably engaged with each other by a pivot element **260.** In some embodiments, the first and second retention members **220A** and **220B** are further associated with one or more tensioning elements **270** to apply force and push apart the first and second retention members **220A** and **220B** such that cranial tissue is retracted away from the burr hole **11.** In some embodiments of the catheter stabilizer **200,** the first and second retention members **220A** and **220B** each include a respective flange **229A** and **229B** (FIG. **6**) to allow a user to grip the retention members **220A** and **220B** with their fingers during insertion and stabilization of the catheter stabilizer **200** within the incision **10.** The flanges **229A** and **229B,** when "pinched" together by a user, allow the first and second retention members **220A** and **220B** to be brought together, as shown in FIG. **8****.** This allows ease of insertion within an incision, with the first and second retention members **220A** and **220B** being pushed apart again to the position shown in FIGS. **5-7** by tensioning element **270** to retract back cranial tissue. As further shown, retention members **220A** and **220B** further include respective concave outer surfaces **222A** and **222B** for engagement with cranial tissue, analogous to concave outer surfaces **122A** and **122B** of the embodiment of FIG. **1****.** Similarly, retention members **220A** and **220B** include respective inner surfaces **224A** and **224B** that collectively define a channel **206** for passage of a catheter. As shown, the retention body **202** further includes an engagement rod **230** for engagement of the occluding arm **204,** and a tail flange **290** (FIG. **7**) defined at an apex between the retention members **220A** and **220B** for additional stabilization of the catheter stabilizer **200** within an incision **10**. In some embodiments, tail flange **290** may further include one or more perforations (not shown) for insertion of a securing member such as securing member **116** (FIG. **1**) to secure catheter stabilizer **200** to a cranium.

Further, in the embodiment of FIGS. **5-8****,** the catheter stabilizer **200** includes the occluding arm **204** that is similar in configuration to the occluding arm **104** of the catheter stabilizer **100** of FIGS. **1-4****,** including a proximal portion **250** and a distal portion **260** defining a free end and a receptacle **242** for insertion of a catheter **20.** As shown in FIG. **5****,** the occluder arm **204** of the catheter stabilizer **200** includes a median portion **255** that defines a "grade" or "z-bend" shape in the occluder arm **204,** allowing the occluder arm **204** to "reach" deeper into the channel **206** defined by the catheter stabilizer **200** for receipt of a catheter. It should be noted that in some embodiments, occluder arm **204** including the "z-bend" shaped median portion **255** can be used with the retention bodies **102, 302, 402** and **502** of FIGS. **1-3** and **9-16** to allow for flush contact of the occluder arm **204** with the cranium, improving how the catheter **20** is inserted.

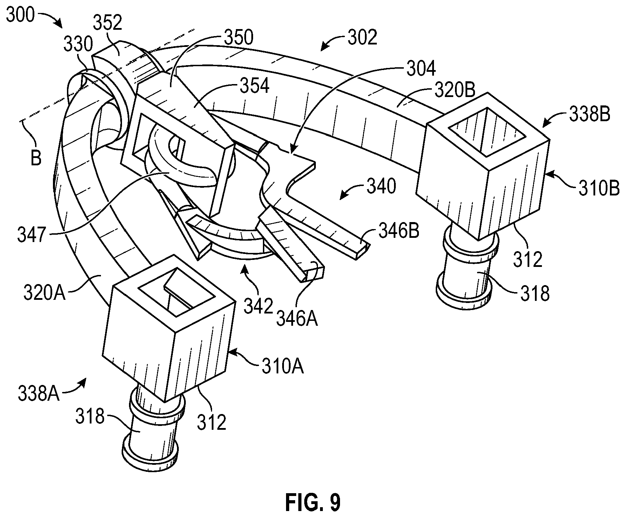

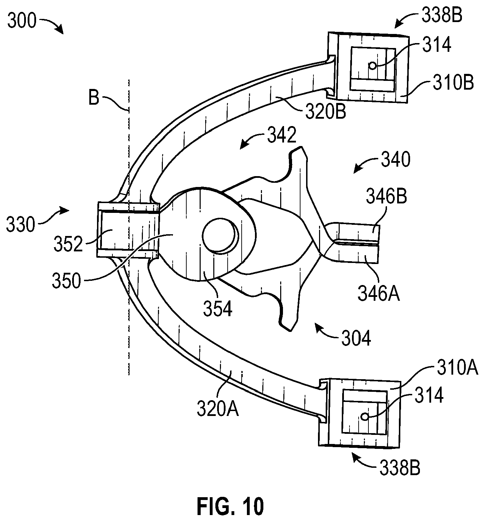

A third embodiment of a catheter stabilizer **300** is shown in FIGS. **9-****11.** As illustrated, the catheter stabilizer **300** includes a retention body **302** for insertion and engagement within the incision **10** (FIG. **17A**) to retain cranial tissue away from the burr hole **11,** the retention body **302** including first and second retention members **320A** and **320B** defining respective distal free ends **338A** and **338B.** In some embodiments the first and second retention members **320A** and **320B** integrally form an arc of the retention body **302.** As shown, an engagement rod **330** for coupling with an occluder arm **304** is defined at an apex of the arc. Similar to previous embodiments, the occluder arm **304** is operable for rotation about an axis **B** defined by the engagement rod **330.** Referring to FIGS. **9** and **10****,** the retention body **302** further includes a first engagement point **310A** and a second engagement point **310B** respectively located at each distal free ends **338A** and **338B** of each retention member **320A** and **320B.** The engagement points **310** each include a surface **312** for contacting the cranium with a perforation **314** formed through each surface **312** for insertion of a securing member (not shown) into the cranium to secure the catheter stabilizer **300** to the cranium. In the present embodiment, each engagement point **310** includes an anchor portion **318** for anchoring the retention body **302** into pilot holes (not shown but analogous to pilot hole **12** of FIG. **17A**) drilled into the cranium.

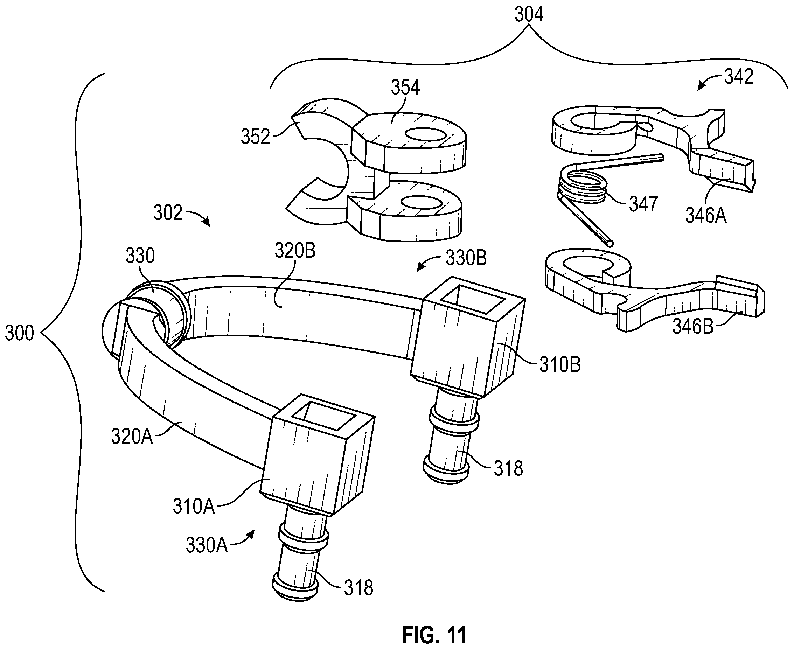

As shown in FIGS. **10** and **11****,** the occluder arm **304** in some embodiments defines a proximal portion **350** and an opposite distal portion **340** defining a free end. The distal portion **350** of the occluder arm **304** includes a receptacle **342** analogous to the respective receptacles **142** and **242** of the catheter stabilizers **100** and **200** and including a first prong **346A** and a second prong **346B.** In some embodiments, receptacle **342** is a clamp assembly. The first and second prongs **346A** and **346B** are associated with the proximal portion **350** of the occluder arm **304** by a tensioning element **347** of the receptacle **342** that forces the first and second prongs **346A** and **346B** to grip and occlude the catheter **20.** As shown, the proximal portion **350** includes a hand **352** to couple the occluder arm **304** to the engagement rod **330** of the retention body **302** and a feedthrough portion **354** for receipt of the receptacle **342.**

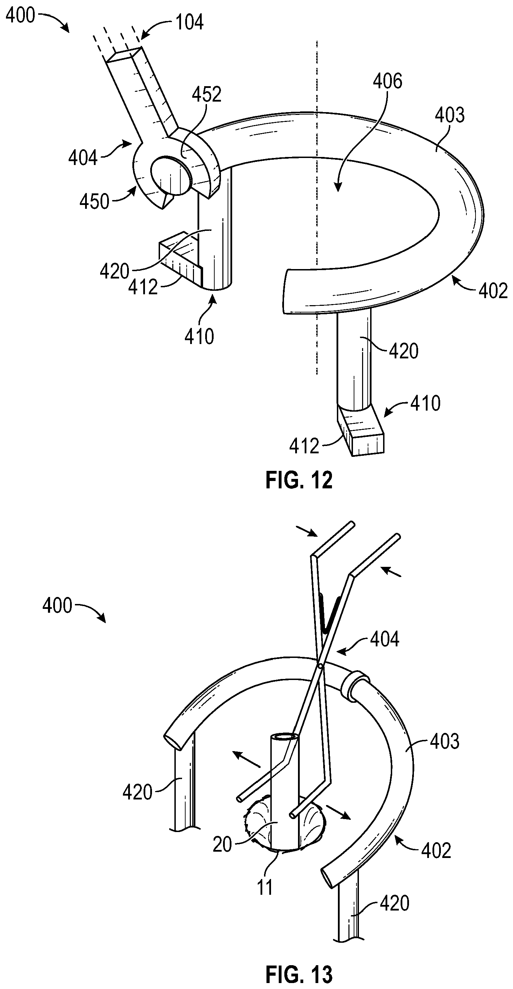

Referring to FIGS. **12** and **13****,** a fourth embodiment of a catheter stabilizer **400** is illustrated. As shown, the catheter stabilizer **400** includes a retention body **402** and an occluding arm **404,** the retention body **402** forming a channel **406.** A distal end (not shown) of occluding arm **404** can be slotted as in the occluding arm of FIG. **4** or can include a clamp assembly as in the occluding arm of FIG. **9****.** A proximal end **450** can include hand portion **452,** similar to embodiments **100, 200** and **300** of FIGS. **4****,** **5****,** and **9****.** In the present embodiment, an engagement rod **403** forms the majority of the retention body **402** such that various embodiments of the occluding arm **404** can be placed anywhere on the engagement rod **403.** In some embodiments, more than one occluding arm **404** can be coupled to the engagement rod **403** in order to stabilize and occlude more than one catheter **20.** In other embodiments, the occluding arm **404** can resemble a "scissor" configuration, as shown in FIG. **13****.** The retention body **402** of the catheter stabilizer **400** can include a plurality of retention members **420** to retract cranial tissue which, in the embodiment shown, can align with an axis defined by the channel **406** and the burr hole **11.** In some embodiments, any number of retention members **420** can be included. In the embodiments of FIGS. **12-13****,** the retention members **420** are each associated with a respective engagement point **430** defining a surface **412** and a perforation (not shown) for engagement with a securing member (not shown).

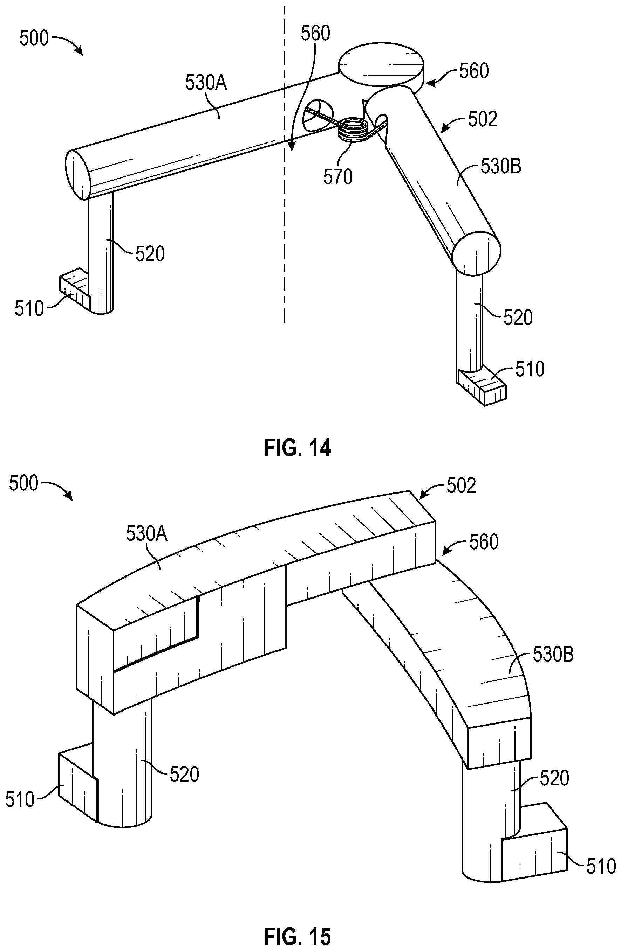

Referring to FIGS. **14-16****,** a fifth embodiment of a catheter stabilizer **500.** As shown, the catheter stabilizer 500 includes a retention body **502,** which in some embodiments, may define a joint or pivot element **560** linking a first engagement rod **530A** and a second engagement rod **530B** with each engagement rod **530** being configured to receive an occluding arm (not shown). Suitable occluding arms for use with retention body **502** can include occluding arms **104** (FIG. **4**), occluding arms **204** (FIG. **5**) and **304** of FIG. **9****.** In operation, the engagement rods **530A** and **530B** are tensioned away from each other by tensioning element **570** to retract back cranial tissue. Similarly to the previous embodiment, the retention body **502** of the catheter stabilizer **500** can include a plurality of retention members **520** to retract cranial tissue which, in the present embodiment, can align with an axis defined by a channel **506** and the burr hole **11.** In some embodiments, any number of retention members **520** can be included. The retention members **520** are each associated with an engagement point **530** defining a surface **512** and a perforation (not shown) for engagement with a securing member (not shown). Referring to FIGS. **15-16****,** in some embodiments the engagement rods **530A** and **530B** can be "wide" enough to both engage an appropriate occluding arm and retract back cranial tissue.

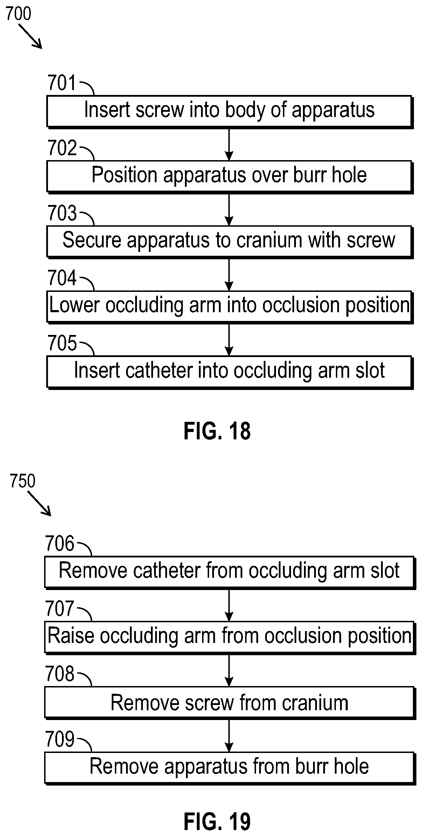

Referring to FIGS. **18** and **19****,** a non-claimed method **700** for insertion of a catheter stabilizer **100** within an incision **10** is illustrated. For ease of review, components of the catheter stabilizer **100** are described with respect to embodiment **100** shown in FIGS. **1-4**; however, various other embodiments are contemplated herein for use with methods **700** and **750,** as discussed above. Photographic images of each step in the method **700** are shown in FIGS. **17A-17E****.** At block **701** (FIG. **17A**), one or more securing members **116** are inserted into the catheter stabilizer **100.** At block **702** (FIG. **17B**), the catheter stabilizer **100** is positioned over a burr hole including a catheter and cranial tissue is retracted away from the burr hole by retention members **120A** and **120B** of catheter stabilizer **100.** At block **703** (FIG. **17C**), the catheter stabilizer **100** is secured to a cranium by the one or more securing members **116.** At block **704** (FIG. **17D**), the occluding arm **104** of the catheter stabilizer **100** is lowered into an "occlusion" position (shown in FIG. **2**) above the burr hole and the channel defined by retention members **120A** and **120B.** At block **705** (FIG. **17E**), the catheter is inserted into the occluding arm **104** and "pinched" shut by the occluding arm **104.**

A non claimed method **750** for subsequent removal of the catheter stabilizer **100** is shown in FIG. **19****.** Photographic images illustrating each step in the method **750** are shown in FIGS. **17G****,** **17J** and **17K****.** At block **706** (FIG. **17G**), the catheter **20** is removed from the occluding arm **104.** At block **707** (FIG. **17J**), the occluding arm **104** is raised away from the "occlusion" position above the burr hole to expose the channel **106.** At block **708** (FIG. **17J**), the one or more securing members **116** are removed or loosened within their pilot holes. At block **709** (FIG. **17K**), the catheter stabilizer **100** is then carefully removed from the incision **10.**

It should be understood from the foregoing that, while particular embodiments have been illustrated and described, various modifications can be made thereto without departing from the scope of the invention as will be apparent to those skilled in the art. Such changes and modifications are within the scope and teachings of this invention as defined in the claims appended hereto.

## Claims

1. A catheter stabilizer apparatus (100), the catheter stabilizer apparatus including;
a retention body (102) including:
one or more retention members (120A, 120B) in association with the retention body (102); and
a channel (106) collectively defined by the one or more retention members (120A, 120B), wherein the channel defines a vertical axis A; and
an occluder arm (104) defining a proximal portion (150) and a distal portion (160), the proximal portion (150) of the occluder arm (104) being pivotably engaged with the retention body (102) such that the occluder arm is operable for being lowered across the channel (106) to an occluding position, and the distal portion (160) of the occluder arm defining a receptacle (142),
wherein the receptacle (142) defines a slotted configuration, and wherein the receptacle (142) is configured to receive a catheter (20) such that fluid flow from the catheter is prevented.

2. The catheter stabilizer apparatus (100) of claim 1, wherein the proximal portion of the occluder arm (104) is pivotably engaged with the retention body (102) such that the occluder arm (104) is rotated about horizontal axis **B** between a peripheral position and the occluding position.

3. The catheter stabilizer apparatus (100) of claim 2, wherein the occluder arm (104) is positioned over the channel (106) perpendicular to horizontal axis **B** and axis **A** defined by the channel (106) when the occluder arm (104) is in the occluding position.

4. The catheter stabilizer apparatus (100) of claim 1, wherein each retention member of the one or more retention members (120A, 120B) defines a concave outer surface (122).

5. The catheter stabilizer apparatus (100) of claim 4, wherein each of the one or more retention members (120A, 120B) defines a flange portion (129A, 129B) located above the concave outer surface (122).

6. The catheter stabilizer apparatus (100) of claim 1, wherein each retention member of the one or more retention members (120A, 120B) defines an inner surface (124A, 124B), wherein the arrangement of inner surfaces of each retention member of the one or more retention members collectively defines the channel (106).

7. The catheter stabilizer apparatus (100) of claim 1, wherein the retention body (102) includes an engagement rod (130) and wherein the engagement rod (130) defines a horizontal axis **B.**

8. The catheter stabilizer apparatus (100) of claim 7, wherein the engagement rod (130) is located at a junction between each of the one or more retention members (120A, 120B).

9. The catheter stabilizer apparatus (100) of claim 7, wherein the occluder arm (104) is removably coupled to the engagement rod (130).

10. The catheter stabilizer apparatus (100) of claim 1, wherein the proximal portion (150) of the occluder arm (104) defines a hand (152), wherein the hand (152) is configured to be removably coupled to the retention body (102).

11. The catheter stabilizer apparatus (100) of claim 1, wherein the retention body (102) includes at least one engagement point (110) for insertion of a securing member (116), optionally wherein the at least one engagement point (110) includes a perforation (113) defined through a surface (112), wherein the perforation (113) is configured to receive a securing member (116).

12. The catheter stabilizer apparatus (100) of claim 1, wherein the retention body (102) further comprises a tensioning element (270, 570) configured for applying tension to the one or more retention members (120A, 120B) such that the one or more retention members are pushed apart.

13. The catheter stabilizer apparatus (100) of claim 1, wherein the occluder arm (104) includes a medial portion such that the distal portion (160) of the occluder arm (104) is located within the channel (106).

14. The catheter stabilizer apparatus (100) of claim 1, wherein the occluder arm (104) and the retention body (102) are comprised of a non-ferromagnetic material.

## Patentansprüche

1. Katheterstabilisierungsvorrichtung (100), wobei die Katheterstabilisierungsvorrichtung Folgendes enthält;
einen Rückhaltekörper (102), der Folgendes enthält:
ein oder mehrere Rückhalteelemente (120A, 120B) in Verbindung mit dem Rückhaltekörper (102); und
einen Kanal (106), der kollektiv durch das eine oder die mehreren Rückhalteelemente (120A, 120B) definiert ist, wobei der Kanal eine vertikale Achse A definiert; und
einen Okklusionsarm (104), der einen proximalen Abschnitt (150) und einen distalen Abschnitt (160) definiert, wobei der proximale Abschnitt (150) des Okklusionsarms (104) schwenkbar mit dem Rückhaltekörper (102) in Eingriff steht, sodass der Okklusionsarm dazu betreibbar ist, über den Kanal (106) in eine Okklusionsposition abgesenkt zu werden, und der distale Abschnitt (160) des Okklusionsarms eine Aufnahme (142) definiert,
wobei die Aufnahme (142) eine geschlitzte Konfiguration definiert und wobei die Aufnahme (142) dazu konfiguriert ist, einen Katheter (20) derart aufzunehmen, dass ein Fluidstrom von dem Katheter verhindert wird.

2. Katheterstabilisierungsvorrichtung (100) nach Anspruch 1, wobei der proximale Abschnitt des Okklusionsarms (104) schwenkbar mit dem Rückhaltekörper (102) in Eingriff steht, sodass der Okklusionsarm (104) um die horizontale Achse **B** zwischen einer peripheren Position und der Okklusionsposition gedreht wird.

3. Katheterstabilisierungsvorrichtung (100) nach Anspruch 2, wobei der Okklusionsarm (104) über dem Kanal (106) senkrecht zu der horizontalen Achse **B** und der durch den Kanal (106) definierten Achse **A** positioniert ist, wenn sich der Okklusionsarm (104) in der Okklusionsposition befindet.

4. Katheterstabilisierungsvorrichtung (100) nach Anspruch 1, wobei jedes Rückhalteelement von dem einen oder den mehreren Rückhalteelementen (120A, 120B) eine konkave Außenoberfläche (122) definiert.

5. Katheterstabilisierungsvorrichtung (100) nach Anspruch 4, wobei jedes von dem einen oder den mehreren Rückhalteelementen (120A, 120B) einen Flanschabschnitt (129A, 129B) definiert, der sich über der konkaven Außenoberfläche (122) befindet.

6. Katheterstabilisierungsvorrichtung (100) nach Anspruch 1, wobei jedes Rückhalteelement von dem einen oder den mehreren Rückhalteelementen (120A, 120B) eine Innenoberfläche (124A, 124B) definiert, wobei die Anordnung von Innenoberflächen jedes Rückhalteelements von dem einen oder den mehreren Rückhalteelementen kollektiv den Kanal (106) definiert.

7. Katheterstabilisierungsvorrichtung (100) nach Anspruch 1, wobei der Rückhaltekörper (102) eine Eingriffsstange (130) enthält und wobei die Eingriffsstange (130) eine horizontale Achse **B** definiert.

8. Katheterstabilisierungsvorrichtung (100) nach Anspruch 7, wobei sich die Eingriffsstange (130) an einer Verbindungsstelle zwischen jedem von dem einen oder den mehreren Rückhalteelementen (120A, 120B) befindet.

9. Katheterstabilisierungsvorrichtung (100) nach Anspruch 7, wobei der Okklusionsarm (104) entfernbar mit der Eingriffsstange (130) gekoppelt ist.

10. Katheterstabilisierungsvorrichtung (100) nach Anspruch 1, wobei der proximale Abschnitt (150) des Okklusionsarms (104) eine Hand (152) definiert, wobei die Hand (152) dazu konfiguriert ist, entfernbar mit dem Rückhaltekörper (102) gekoppelt zu sein.

11. Katheterstabilisierungsvorrichtung (100) nach Anspruch 1, wobei der Rückhaltekörper (102) mindestens einen Eingriffspunkt (110) zum Einführen eines Sicherungselements (116) enthält, wobei der mindestens eine Eingriffspunkt (110) optional eine Perforation (113) enthält, die durch eine Oberfläche (112) definiert ist, wobei die Perforation (113) dazu konfiguriert ist, ein Sicherungselement (116) aufzunehmen.

12. Katheterstabilisierungsvorrichtung (100) nach Anspruch 1, wobei der Rückhaltekörper (102) ferner ein Spannbauteil (270, 570) umfasst, das zum Aufbringen von Spannung auf das eine oder die mehreren Rückhalteelemente (120A, 120B) konfiguriert ist, sodass das eine oder die mehreren Rückhalteelemente auseinandergedrückt werden.

13. Katheterstabilisierungsvorrichtung (100) nach Anspruch 1, wobei der Okklusionsarm (104) einen medialen Abschnitt enthält, sodass sich der distale Abschnitt (160) des Okklusionsarms (104) innerhalb des Kanals (106) befindet.

14. Katheterstabilisierungsvorrichtung (100) nach Anspruch 1, wobei der Okklusionsarm (104) und der Rückhaltekörper (102) aus einem nicht ferromagnetischen Material bestehen.

## Revendications

1. Appareil de stabilisation de cathéter (100), l'appareil de stabilisation de cathéter comprenant :
un corps de retenue (102) comprenant :
un ou plusieurs éléments de retenue (120A, 120B) en association avec le corps de retenue (102) ; et
un canal (106) défini collectivement par lesdits un ou plusieurs éléments de retenue (120A, 120B), le canal définissant un axe vertical A ; et
un bras d'occlusion (104) définissant une partie proximale (150) et une partie distale (160), la partie proximale (150) du bras d'occlusion (104) étant mise en prise de manière pivotante avec le corps de retenue (102) de sorte que le bras d'occlusion puisse être actionné de manière à être abaissé à travers le canal (106) jusqu'à une position d'occlusion, et la partie distale (160) du bras d'occlusion définissant un réceptacle (142),
ledit réceptacle (142) définissant une configuration fendue, et ledit réceptacle (142) étant conçu pour recevoir un cathéter (20) de sorte que l'écoulement de fluide à partir du cathéter soit empêché.

2. Appareil de stabilisation de cathéter (100) selon la revendication 1, ladite partie proximale du bras d'occlusion (104) étant mise en prise de manière pivotante avec le corps de retenue (102) de sorte que le bras d'occlusion (104) tourne autour de l'axe horizontal B entre une position périphérique et la position d'occlusion.

3. Appareil de stabilisation de cathéter (100) selon la revendication 2, ledit bras d'occlusion (104) étant positionné sur le canal (106) perpendiculairement à l'axe horizontal B et à l'axe A défini par le canal (106) lorsque le bras d'occlusion (104) se trouve dans la position d'occlusion.

4. Appareil de stabilisation de cathéter (100) selon la revendication 1, chaque élément de retenue desdits un ou plusieurs éléments de retenue (120A, 120B) définissant une surface extérieure concave (122).

5. Appareil de stabilisation de cathéter (100) selon la revendication 4, chacun desdits un ou plusieurs éléments de retenue (120A, 120B) définissant une partie de bride (129A, 129B) située au-dessus de la surface extérieure concave (122).

6. Appareil de stabilisation de cathéter (100) selon la revendication 1, chaque élément de retenue desdits un ou plusieurs éléments de retenue (120A, 120B) définissant une surface intérieure (124A, 124B), ledit agencement des surfaces intérieures de chaque élément de retenue desdits un ou plusieurs éléments de retenue définissant collectivement le canal (106).

7. Appareil de stabilisation de cathéter (100) selon la revendication 1, ledit corps de retenue (102) comprenant une tige de mise en prise (130) et ladite tige de mise en prise (130) définissant un axe horizontal **B.**

8. Appareil de stabilisation de cathéter (100) selon la revendication 7, ladite tige de mise en prise (130) étant située au niveau d'une jonction entre chacun desdits un ou plusieurs éléments de retenue (120A, 120B).

9. Appareil de stabilisation de cathéter (100) selon la revendication 7, ledit bras d'occlusion (104) étant couplé de manière amovible à la tige de mise en prise (130).

10. Appareil de stabilisation de cathéter (100) selon la revendication 1, ladite partie proximale (150) du bras d'occlusion (104) définissant une main (152), ladite main (152) étant conçue pour être couplée de manière amovible au corps de retenue (102).

11. Appareil de stabilisation de cathéter (100) selon la revendication 1, ledit corps de retenue (102) comprend au moins un point de mise en prise (110) pour l'insertion d'un élément de fixation (116), éventuellement ledit au moins un point de mise en prise (110) comprenant une perforation (113) définie à travers une surface (112), ladite perforation (113) étant conçue pour recevoir un élément de fixation (116).

12. Appareil de stabilisation de cathéter (100) selon la revendication 1, ledit corps de retenue (102) comprenant en outre un élément de mise en tension (270, 570) conçu pour appliquer une tension sur lesdits un ou plusieurs éléments de retenue (120A, 120B) de sorte que lesdits un ou plusieurs éléments de retenue soient écartés.

13. Appareil de stabilisation de cathéter (100) selon la revendication 1, ledit bras d'occlusion (104) comprenant une partie médiane de sorte que la partie distale (160) du bras d'occlusion (104) soit située à l'intérieur du canal (106).

14. Appareil de stabilisation de cathéter (100) selon la revendication 1, ledit bras d'occlusion (104) et ledit corps de retenue (102) étant composés d'un matériau non ferromagnétique.
